# EUROPÄISCHE PATENTSCHRIFT

(11) **EP 1 828 767 B1**
(45) Veröffentlichungstag und Bekanntmachung des Hinweises auf die Patenterteilung: **03.03.2010**
(21) Anmeldenummer: 05811350.7
(22) Anmeldetag: 30.11.2005
(51) Int. Cl.: G01N 33/15, G01N 3/02

(54) **EINRICHTUNG ZUM TESTEN DER BRUCHFESTIGKEIT VON TABLETTEN**
APPLIANCE FOR TESTING THE FRACTURE RESISTANCE OF TABLETS
ENSEMBLE POUR TESTER LA RESISTANCE A LA FRACTURE DE COMPRIMES

(30) Priorität: 13.12.2004 DE 102004059976
(43) Veröffentlichungstag der Anmeldung: 05.09.2007
(73) Patentinhaber: ERWEKA GmbH, 63150 Heusenstamm (DE)
(72) Erfinder: BOZKURT, Levent, Erweka GmbH, 63150 Heusenstamm (DE); MÜLLER, Werner G., Erweka GmbH, 63150 Heusenstamm (DE)
(74) Vertreter: Meyer-Dulheuer, Karl-Hermann
(86) Internationale Anmeldenummer: PCT/EP2005/012780
(87) Internationale Veröffentlichungsnummer: WO 2006/063685

(56) Entgegenhaltungen:
- DE-U1- 8 402 581
- DE-U1- 8 516 163
- US-A- 5 555 768

## Beschreibung

Die Erfindung betrifft ein Tablettentestgerät zum Testen von Oblong-Tabletten, Dragees oder ähnlich geformten Tabletten, insbesondere Vermessen und Bruchtesten derartiger Tabletten, in entsprechenden Teststationen eine Transporteinrichtung, die eine Basis mit einer Auflagefläche, auf der die zu testende Tablette abzulegen ist, sowie ein Transportmittel, das die auf der Auflagefläche abgelegten Tabletten durch die Teststationen transportiert, wobei die Bruchfestigkeitsmeßeinrichtung zum Testen der die Bruchfestigkeit der Tabletten einen bewegbaren Bruchbacken und einen Gegenbacken, um die auf der Auflageeinrichtung abgelegte Tablette zwischen sich zu Bruch zu bringen, eine- Antriebseinrichtung für den Bruchbacken und eine Meßeinrichtung zur Messung der Kraft umfaßt, die für den Bruch der Tablette erforderlich ist.

Aus DE 84 02 581 U1 ist ein Tablettentestgerät mit einer Transporteinrichtung bekannt, bei dem die Tabletten von einer Zufuhreinrichtung auf eine Transporteinrichtung mit einem Rechen gelangen, der mehrere Gabeln aufweist, die die Tabletten auf einer Transportbahn weiterbewegen. Eine Bewegungseinrichtung für den Rechen transportiert den Rechen um eine Strecke in Richtung der Transportrichtung, hebt dann den Rechen an und führt ihn in angehobenem Zustand entgegen der Transportrichtung um eine entsprechende Strecke wieder zurück. Anschließend wird der Rechen abgesenkt und erneut um eine Transportstrecke weiterbewegt. Entlang der Bewegungsbahn des Rechens sind mehrere Teststationen, nämlich eine Waage, eine Teststation für die Dickenmessung und eine Teststation für den Bruchtest vorgesehen. Das bekannte Tablettentestgerät ist für runde oder kugelförmige Tabletten geeignet, weniger jedoch für sogenannte Oblong-Tabletten und ähnlich geformte Tabletten, die eine im wesentlichen ovale Form in Draufsicht, einen flachen Steg an ihrem Umfang sowie eine gewölbte Ober- und Unterseite aufweisen können. Derartige Tabletten bereiten auf dem bekannten Gerät Schwierigkeiten. Sie können in verschiedenen Teststationen, wie der Dickenmeßstation, der Durchmessermeßstation und der Station zur Bruchhärteprüfung nicht immer in der erforderlichen Weise ausgerichtet werden.

Tablettentestgeräte mit mehreren Teststationen für Tabletten und einer Transporteinrichtung, die die Tabletten von einer Teststation zur nächsten Teststation transportiert, sind ferner aus der DE 35 60 440 A1 bekannt. Bei den Teststationen kann es sich beispielsweise um eine Wägestation, in der die Tabletten gewogen werden, um eine Dickenmeßstation, in der die Dicke der Tabletten gemessen wird, und eine Station handeln, bei der eine Einrichtung zum Testen der Bruchfestigkeit von Tabletten vorgesehen ist. Als Transporteinrichtung kann, wie in der DE 35 60 440 A1 gezeigt ist, ein sogenannter Transportrechen verwendet werden, der die Tabletten entlang einer linearen Bahn transportiert. Was Bruchfestigkeitsmeßeinrichtungen betrifft, besteht eines der Probleme darin, daß die Tabletten exakt an der gleichen Stelle auf der Auflageeinrichtung in der Bruchfestigkeitsmeßeinrichtung angeordnet werden müssen und daß die Tabletten, die eine längliche Form mit einem großen Durchmesser und einem kleinen Durchmesser haben, sogenannte Oblongs, immer mit der großen Längsachse senkrecht zu der Bewegung des Bruckbackens angeordnet sein müssen, um definiert Werte für die Bruchfestigkeit erhalten zu können. Auch bereiten Bruchreste der zerbrochenen Tablette an den Backen der Bruchfestigkeitsmeßeinrichtung und auf der Auflageeinrichtung Probleme, wenn diese Bruchreste nicht vollständig nach jedem Bruchvorgang entfernt werden, weil derartige Bruchreste die Genauigkeit der nachfolgenden Messung nachteilig beeinflussen können.

Ein weiteres gattungsgemäßes Gerät wird in US 5 555 768 beschreiben.

Es ist Aufgabe der Erfindung, eine Einrichtung zum Testen der Bruchfestigkeit von Tabletten und ein Tablettentestgerät mit einer Bruchfestigkeitsmesseinrichtung der eingangs genannten Art bereit zu stellen, wobei die zu testenden Tabletten unabhängig von deren Form immer in der gleichen Lage bezüglich der Backen der Bruchfestigkeitsmesseinrichtung angeordnet sind, so daß die Bruchfestigkeitsmessung mit hoher Genauigkeit durchgeführt werden kann.

Zur Lösung dieser Aufgabe ist die erfindungsgemäße Einrichtung dadurch gekennzeichnet, dass in Transportrichtung hinter der Bruchfestigkeitsmesseinrichtung ein beweglicher Anschlag zum Aufhalten der Tablette in der Bruchfestigkeitsmesseinrichtung angeordnet ist, und dass an dem Transportmittel ein Positionierungswerkzeug angeordnet ist, das bis auf einen Abstand, der von einer Breitenabmessung der Tablette bestimmt ist, zu dem Anschlag hin bewegbar ist. Die Tablette wird somit zwischen dem beweglichen Anschlag und dem Positionierwerkzeug in einer wohl definierten Lage gehalten, so dass bei der Bruchfestigkeitsmessung reproduzierbare Verhältnisse bezüglich der Lage der Tablette zu den Bruchbacken gegeben sind.

Eine vorteilhafte Ausgestaltung des erfindungsgemäßen Tablettentestgeräts ist dadurch gekennzeichnet, dass das Transportmittel ein Transportrechen ist. Damit ergibt sich in vorteilhafter Weise die Möglichkeit, das Positionierwerkzeug an dem Transportrechen zu befestigen, so dass es über den Bewegungsmechanismus des Transportrechens steuerbar ist.

Eine weitere vorteilhafte Ausgestaltung des erfindungsgemäßen Tablettentestgeräts ist dadurch gekennzeichnet, dass der bewegliche Anschlag eine unterhalb der Auflagefläche schwenkbar gelagerte Klappe aufweist, die mit einer Antriebseinrichtung verbunden ist, die in der Lage ist, die Klappe zwischen einer Ruhestellung, in der die Klappe unterhalb der Auflagefläche liegt, und einer Anschlagstellungen, in der sie den Anschlag bildet, indem sie über die Auflagefläche hinausragt, hin- und her zu bewegen. Mit einer derartigen Klappe kann in vorteilhafter Weise der bewegliche Anschlag verwirklicht werden, wobei die Klappe auch aus der Bahn der Transporteinrichtung heraus bewegt werden kann, wenn das Transportmittel die Tabletten weiter befördert und insbesondere die bereits zerbrochene Tablette zum Abfall befördert.

Eine weitere vorteilhafte Ausgestaltung des erfindungsgemäßen Tablettentestgeräts ist dadurch gekennzeichnet, dass das Positionierungswerkzeug lösbar an der Transporteinrichtung befestigt ist. Damit kann das Positionierwerkzeug je nach der zu testenden Tablettenart leicht ausgetauscht werden.

Eine weitere vorteilhafte Ausgestaltung des erfindungsgemäßen Tablettentestgeräts ist dadurch gekennzeichnet, dass das Positionierungswerkzeug in Wirkrichtung des Bruchbackens verschiebbar an der Transporteinrichtung gelagert ist. Damit wird eine sichere Führung der Tablette zu der definierten Endlage, in der der Bruchtest durchgeführt wird, bewirkt auch dann, wenn die Tablette nach dem Transport außermittig an dem Anschlag angekommen sein sollte.

Eine weitere vorteilhafte Ausgestaltung des erfindungsgemäßen Tablettentestgeräts ist dadurch gekennzeichnet, dass das Positionierungswerkzeug federnd gegen die Wirkrichtung des Bruchbackens vorgespannt und von einem mit der Bewegungseinrichtung des Bruchbackens gekoppelten Stössel in Wirkrichtung des Bruchbackens verschiebbar ist. Durch die federnde Lagerung des Positionierungswerkzeugs wandert das Positionierwerkzeug quasi mit der Bewegung des Bruchbackens mit und sorgt dabei in vorteilhafter Weise für eine verbesserte Positionierung der Tablette in der Soll-Lage für den Bruchtest praktisch unabhängig von der ursprünglichen Lage der Tablette in der Bruchfestigkeitsmessstation. Außerdem ist für die Bewegung des Positionierwerkzeuges kein separater Antrieb erforderlich. Durch die federnde Lagerung des Positionierungswerkzeugs wird ferner erreicht, dass dieses in seine Ausgangslage zurückkehrt, wenn der Bruchbacken zurückgefahren wird.

Eine weitere vorteilhafte Ausgestaltung des erfindungsgemäßen Tablettentestgeräts ist dadurch gekennzeichnet, dass das Positionierungswerkzeug einen Vorsprung an dem Transportmittel aufweist, der von der Auflagefläche bis auf eine Höhe über der Auflagefläche reicht, die höher ist als eine Höhenabmessung der Tablette. Damit ist in vorteilhafter Weise sichergestellt, dass die Tablette von dem Positionierungswerkzeug tatsächlich auch festgehalten wird.

Eine weitere vorteilhafte Ausgestaltung des erfindungsgemäßen Tablettentestgeräts ist dadurch gekennzeichnet, dass der Vorsprung des Transportmittels in Wirkrichtung des Bruchbackens eine Breitenabmessung größer als die Hälfte der Länge oder der Breite der Tablette hat. Diese Dimensionierung ist vorteilhaft im Hinblick auf eine sichere Positionierung der Tablette.

Eine weitere vorteilhafte Ausgestaltung des erfindungsgemäßen Tablettentestgeräts ist dadurch gekennzeichnet, dass das Positionierungswerkzeug an seiner dem Anschlag zugewandten Seite eine Nase aufweist, deren untere Begrenzungsfläche bei einer Stelle an einer Stirnfläche der Vorsprungs liegt, deren Höhe über der Auflagefläche durch die Dickenabmessung der Tablette bestimmt ist. Eine derartige Nase hat sich insbesondere bei Dragees als vorteilhaft erwiesen, die dazu neigen, in der Bruchfestigkeitsmessstation sich beim Bruchtest aufzustellen. Die Nase dient in vorteilhafter Weise dazu, das Dragee während des Bruchtests am Aufstellen zu hindern und damit auch diesen Fehler bei der Bruchfestigkeitsmessung zu vermeiden.

Ausführungsbeispiele der Erfindung werden nun anhand der beiliegenden Zeichnungen beschrieben. Es zeigen:
Figur 1 eine perspektivische Ansicht einer Transportvorrichtung mit einer Positionierungseinrichtung nach einem Ausführungsbeispiel der Erfindung;
Figur 2 eine Draufsicht auf eine Bruchfestigkeitstesteinrichtung mit der Positionierungseinrichtung nach Figur 1;
Figur 3A eine schematische Seitenansicht Bruchfestigkeitstesteinrichtung mit der Positionierungseinrichtung nach Figur 1, wobei die Positionierungseinrichtung in ihrer Ausgangsposition gezeigt ist;
Figur 3B eine schematische Seitenansicht Bruchfestigkeitstesteinrichtung mit der Positionierungseinrichtung nach Figur 1, wobei die Positionierungseinrichtung in ihrer Positionierungsposition gezeigt ist;
Figur 4 eine Draufsicht auf eine Bruchfestigkeitstesteinrichtung mit der Positionierungseinrichtung nach einem weiteren Ausführungsbeispiel der Erfindung, wobei die Positionierungseinrichtung in ihrer Ausgangsposition gezeigt ist;
Figur 5 eine Draufsicht auf eine Bruchfestigkeitstesteinrichtung mit der Positionierungseinrichtung nach Figur 4, wobei die Positionierungseinrichtung in ihrer Positionierungsposition gezeigt ist; und
Figur 6 eine perspektivische Ansicht einer Transportvorrichtung mit einer Positionierungseinrichtung nach einem weiteren Ausführungsbeispiel der Erfindung.

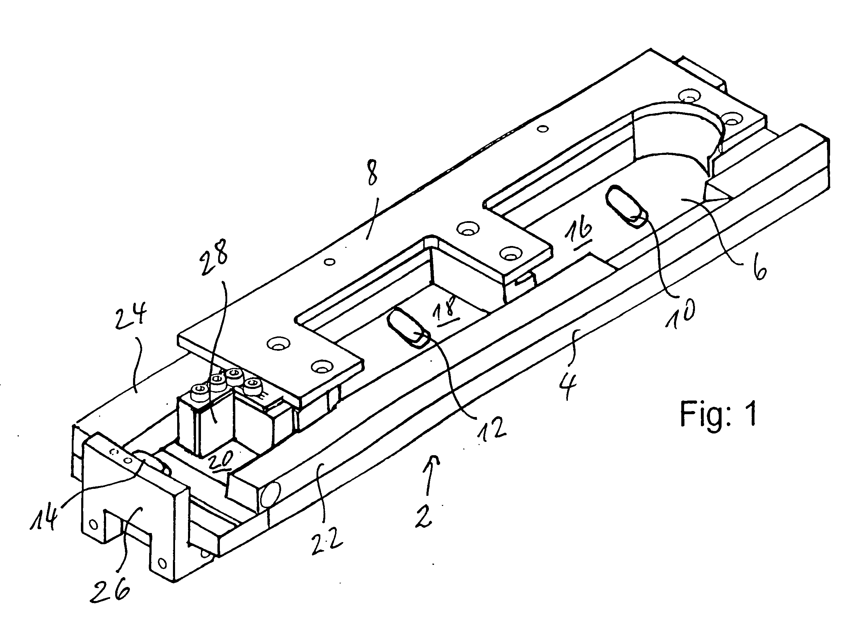

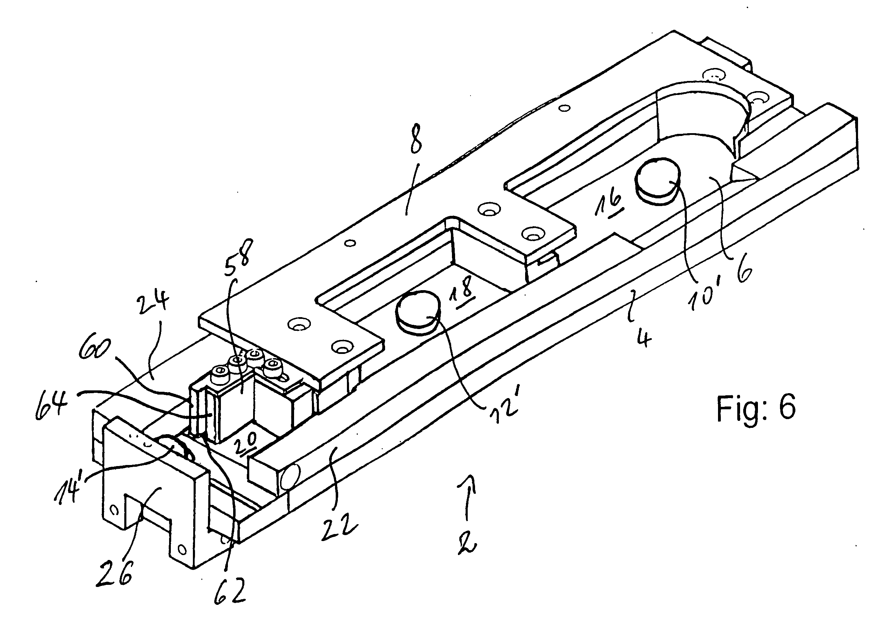

In den Figuren 1 und 6 ist schematisch eine Transporteinrichtung 2 eines Tabletten-Testgeräts gezeigt, wie es beispielsweise in einer älteren Bauweise in der DE 35 60 440 A1 gezeigt ist. Die Transporteinrichtung 2 umfaßt eine Basis 4 mit einer Auflagefläche 6 und einen Transportrechen 8, der zum Transport von Oblong-Tabletten 10, 12, 14 gemäß Figur 1 oder Dregee-Tabletten 10', 12', 14' gemäß Figur 6. Die Tabletten 10, 12, 14; 10', 12', 14' liegen respektive in einer Aufgabestation 16, in die die Tabletten von einem Vorratsbehälter gelangen, einer Dickenmessstation 18, in der ein Dickenmessungs-Sensor von oben an die Tablette herangefahren wird und einer Bruchfestigkeitsmessstation 20, in der die Bruchhärte der Tabletten gemessen wird. Die Transporteinrichtung 2 transportiert die Tabletten von der Aufgabestation 16 über die Meßstationen 18, 20 zur Entsorgung.

Der Transportrechen 8 bewegt sich in einer abgesenkten Position, wie in den Figuren 1 und 6 gezeigt ist, in Transportrichtung, um beispielsweise die Tablette 10 in die Position zu bringen, in der die Tablette 12 dargestellt ist. Durch eine weitere Bewegung des Transportrechens 8 wird die betreffende Tablette dann in die Bruchfestigkeitsmessstation 20 eingebracht, wie durch die Tablette 14 dargestellt ist. Am Ende seiner Bewegungsbahn wird der Transportrechen 8 angehoben und entgegen der Transportrichtung zurück bewegt.

Gemäß den Figuren 1 und 6 sind seitlich über der Basis 4 zwei Führungsstäbe 22, 24 angeordnet, von denen der Führungsstab 24 an seinem vorderen Ende (links in Figur 1) auch als Gegenbacken beim Bruchfestigkeitstest dient. In Transportrichtung (von rechts nach links in Figur 1) hinter der Bruchfestigkeitsmesseinrichtung 20 ist eine drehbar gelagerte Klappe 26 als Anschlag zum Aufhalten der Tablette 14 in der Bruchfestigkeitsmessstation 20 angeordnet. An dem Transportrechen 8 ist ein Positionierwerkzeug 28 angeordnet, das bis auf einen Abstand, der von der Balkenabmessung der Tableite bestimmt ist, zu der Klappe 26 hin bewegbar ist. Das Positionierwerkzeug 28 ist mit Schauben lösbar an dem Transportrechen 8 befestigt, wie in Figur 1 gezeigt ist.

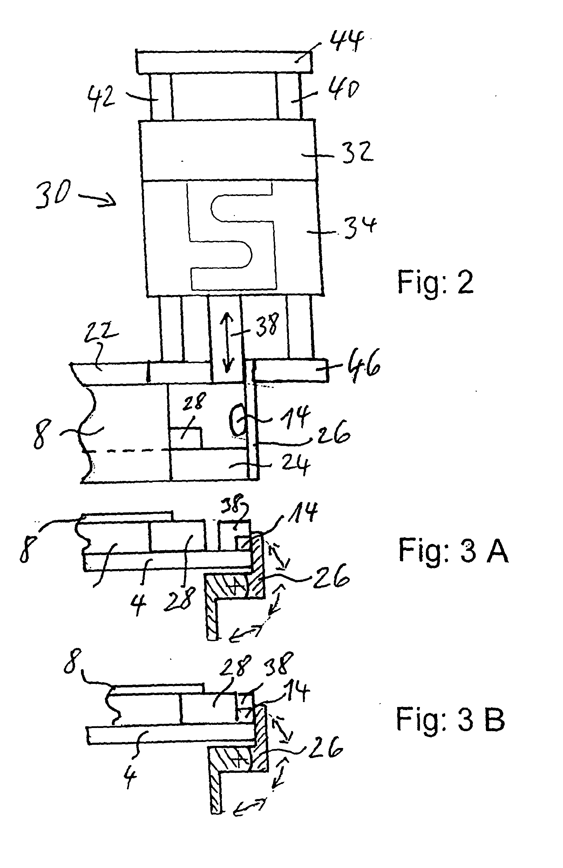

In Figur 2 ist die Bruchfestigkeitsmesseinrichtung 30 gezeigt, die einen Antrieb 32, eine Kraftmessdose 34 und einen Bruchbacken 38 aufweist. Der Antrieb 32 und die Kraftmessdose 34 sind als Schlitten auf Führungsstangen 40, 42 in Richtung des Doppelpfeils in Figur 2 bewegbar, wobei die Führungsstangen 40, 42 in einem Rahmen 44, 46 angeordnet sind.

Die Figuren 3A und 3B zeigen schematisch eine Seitenansicht der Bruchfestigkeitsmesseinrichtung, wobei nur die zur Erläuterung erforderlichen Teile der Einrichtung dargestellt sind. Figur 3A zeigt die Lage der Teile der Einrichtung, wenn die Tablette 14 von der Klappe 26 gehalten wird, wobei das Positionierungswerkzeug 28 sich in der Ruhestellung befindet, in der es noch nicht an der Tablette 14 angreift. Die Klappe 26 ist sowohl in ihrer nach oben geklappten Arbeitsposition als auch in ihrer nach unten geklappten Ruheposition dargestellt. Die Klappe 26 ist unterhalb der Auflagefläche 6 schwenkbar gelagert und ist mit einer Antriebseinrichtung verbunden, die in der Lage ist, die Klappe 26 zwischen einer Ruhestellung, in der die Klappe 26 unterhalb der Auflagefläche 6, liegt, und einer Anschlagstellungen, in der sie den Anschlag bildet, indem sie über die Auflagefläche 6 hinausragt, hin- und her zu bewegen.

Figur 3B zeigt die Position der Teile der Einrichtung, wenn das Positionierungswerkzeug 28 die Tablette zwischen sich und der Klappe 26 festhält, wobei das Positionierungswerkzeug 28 bis auf die Breite der Tablette 14 zu der Klappe 26 hin bewegt worden ist.

Das in den Figuren 2 und 3 gezeigte Ausführungsbeispiel der Bruchfestigkeitsmesseinrichtung 30 arbeitet wie folgt. Der Transportrechen 8 transportiert die Tabletten bis zu der Klappe 26 und fährt wieder in die Ausgangsstellung zurück. Die Klappe 26 schwenkt immer vor dem Transportrechen 8 hoch und dient als Anschlag für die Tabletten. Der Transportrechen 8 fährt in Abhängigkeit der Tablettengröße bzw. Oblongbreite die Tabletten bis zu der Klappe 26 heran, sodass die Tablette zwischen der Klappe 26 und dem Positionierungswerkzeug 28 noch passt. Die Tablette wird nun mit dem Bruchbacken nach vorne in die Lücke zwischen der Klappe 26 und dem Positionierungswerkzeug 28 gefördert und gebrochen. Nach dem Bruch fährt der Transportrechen 8 in die Ausgangsstellung zurück und danach wird nächste Transportvorgang eingeleitet.

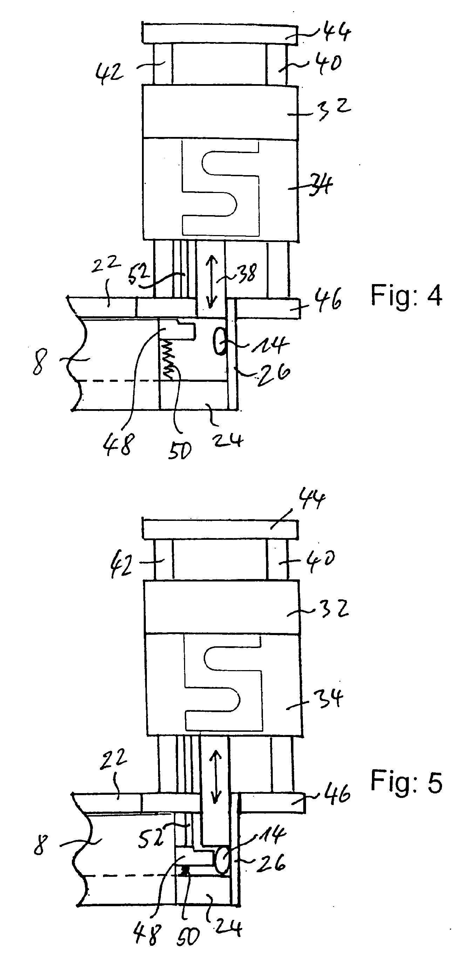

Die Figuren 4 und 5 zeigen ein weiteres Ausführungsbeispiel der Bruchtestigkeitsmesseinrichtung, wobei das Positionierungswerkzeug 48 in Wirkrichtung des Bruchbackens 38 verschiebbar an dem Transportrechen gelagert ist. Das Positionierungswerkzeug 48 ist dabei mit Hilfe einer Feder 50 federnd gegen die Wirkrichtung des Bruchbackens 38 vorgespannt und wird von einem mit der Bewegungseinrichtung der Bruchbackens 38 gekoppelten Stössel 52 in Wirkrichtung des Bruchbackens 38 verschoben.

In Figur 4 ist die Ausgangslage des Positionierungswerkzeuges 48 und in Figur 5 die Arbeitsstellung des Positionierungswerkzeuges 48 gezeigt, in der die Tablette 14 zwischen dem Positionierungswerkzeug und der Klappe 26 festgehalten wird.

Diese Ausführungsform der Bruchfestigkeitsmesseinrichtung arbeitet wie folgt: Der Transportrechen 8 transportiert die Tabletten bis zu der Klappe 26 und fährt wieder in die Ausgangsstellung zurück. Die Klappe 26 schwenkt immer vor dem Transportrechen 8 hoch und dient als Anschlag für die Tabletten. Der Transportrechen 8 fährt in Abhängigkeit der Tablettengröße bzw. Oblongbreite die Tabletten bis zu der Klappe 26 heran, so dass die Oblong-Tablette zwischen der Klappe 26 und dem Positionierungswerkzeug 48 noch passt. Das Positionierungswerkzeug 48 wird von dem Stössel 52 mitgenommen, wenn der Bruchbacken 38 nach vorne fährt. Die Tablette wird zwischen der Klappe 26 und dem Positionierungswerkzeug 48 vom Bruchbacken 38 nach vorne bewegt und gebrochen. Nach dem Bruch fährt der Transportrechen 8 in die Ausgangsstellung zurück und der nächste Transportvorgang wird eingeleitet.

Figur 6 zeigt wiederum eine Transporteinrichtung 2 wie Figur 1 für Dragee-Tabletten 10', 12', 14', wobei in diesem Ausführungsbeispiel das Positionierungswerkzeug 58 an seiner der Klappe 26 als Anschlag zugewandten Seite eine Nase 60 aufweist, deren untere Begrenzungsfläche 62 an einer Stelle an einer Stirnfläche 64 des Positionierungswerkzeuges 58 liegt, deren Höhe über der Auflagefläche 6 durch die Dickenabmessung der Tablette, insbesondere der Drageetablette 14' bestimmt ist.

Dragees neigen dazu, sich während eines Bruchvorganges nach oben zu drehen und sich aufzustellen. Um dies zu verhindern, werden sie von oben mit der Nase 60 als Niederhalter unten gehalten. Der Transportrechen 8 transportiert die Tabletten 10', 12' 14' bis zu der Klappe 26 und fährt wieder in die Ausgangsstellung zurück. Der Transportrechen 8 fährt in Abhängigkeit der Tablettengröße die Tabletten bis zu der Klappe 26 heran, sodass die Tablette 14' zwischen der Klappe 26 und dem Positionierungswerkzeug 58 noch passt. Die Tablette 14' wird nun mit dem Bruchbacken 38 nach vorne unter die Nase 60 des Positionierungswerkzeugs 58 geschoben und gebrochen. Während des Bruchvorgangs wird die Tablette 14' von dem Positionierungswerkzeug 58 unten gehalten. Nach dem Bruch fahren alle Stationen in die Ausgangsstellung zurück und werden gereinigt.

Bei den gezeigten Ausführungsbeispielen weist das Positionierungswerkzeug 28; 48; 58 einen Vorsprung an dem Transportrechen 8 auf, der von der Auflagefläche 6 bis auf eine Höhe über der Auflagefläche 6 reicht, die höher ist als eine Höhenabmessung der Tablette 10, 12, 14 bzw. 10', 12', 14'. Ferner hat der Vorsprung an dem Transportrechen 8 in Wirkrichtung des Bruchbackens 38 eine Breitenabmessung, die größer ist als die Hälfte der Länge oder der Breite der Tablette.

## Patentansprüche

1. Tablettentestgerät zum Testen von Oblong-Tabletten, Dragees oder ähnlich geformten Tabletten, insbesondere Vermessen und Bruchtesten derartiger Tabletten, in entsprechenden Teststationen (18, 20) einer Transporteinrichtung (2), die eine Basis (4) mit einer Auflagefläche (6), auf der die zu testenden Tabletten abzulegen ist, sowie ein Transportmittel, zum Transport der auf der Auflagefläche abgelegten Tabletten durch die Teststationen (18, 20), umfasst, wobei die Bruchfestigkeitsmesseinrichtung zum Testen der die Bruchfestigkeit der Tabletten einen bewegbaren Bruchbacken (38) und einen Gegenbacken (24), um die auf der Auflagefläche (6) abgelegte Tablette zwischen sich zu Bruch zu bringen, eine Antriebseinrichtung (32) für den Bruchbacken (38) und eine Messeinrichtung (34) zur Messung der Kraft umfaßt, die für den Bruch der Tablette erforderlich ist, **dadurch gekennzeichnet, dass**
in Transportrichtung (2) hinter der Bruchfestigkeitsmesseinrichtung (30) ein beweglicher Anschlag (26) zum Aufhalten der Tablette in der Bruchfestigkeitsmesseinrichtung (30) angeordnet ist, und dass
an dem Transportmittel (8) ein Positionierungswerkzeug (28; 48; 58) angeordnet ist, das bis auf einen Abstand, der von einer Breitenabmessung der Tablette bestimmt ist, zu dem Anschlag (26) hin bewegbar ist

2. Tablettentestgerät nach Anspruch 1, **dadurch gekennzeichnet, dass** das Transportmittel (8) ein Transportrechen ist.

3. Tablettentestgerät nach Anspruch 1 oder 2, **dadurch gekennzeichnet, dass** der bewegliche Anschlag eine unterhalb der Auflagefläche (6) schwenkbar gelagerte Klappe (26) aufweist, die mit einer Antriebseinrichtung verbunden ist, die in der Lage ist, die Klappe (26) zwischen einer Ruhestellung, in der die Klappe (26) unterhalb der Auflagefläche (6), liegt, und einer Anschlagstellungen, in der sie den Anschlag bildet, indem sie über die Auflagefläche hinausragt, hin- und her zu bewegen.

4. Tablettentestgerät nach Anspruch 1, **dadurch gekennzeichnet, dass** das Positionierungswerkzeug (28; 48; 58) lösbar an dem Transportmittel (8) befestigt ist.

5. Tablettentestgerät nach Anspruch 1, **dadurch gekennzeichnet, dass** das Positionierungswerkzeug (48) in Wirkrichtung des Bruchbackens (38) verschiebbar an dem Transportmittel (8) gelagert ist.

6. Tablettentestgerät nach Anspruch 1, **dadurch gekennzeichnet, dass** das Positionierungswerkzeug (48) federnd gegen die Wirkrichtung des Bruchbackens (38) vorgespannt und von einem mit der Bewegungseinrichtung des Bruchbackens gekoppelten Stössel (52) in Wirkrichtung des Bruchbackens (38) verschiebbar ist.

7. Tablettentestgerät nach Anspruch 1, **dadurch gekennzeichnet, dass** das Positionierungswerkzeug (28; 48; 58) einen Vorsprung an dem Transportmittel (8) aufweist, der von der Auflagefläche (6) bis auf eine Höhe über der Auflagefläche (6) reicht, die höher ist als eine Höhenabmessung der Tablette (10, 12, 14; 10', 12', 14').

8. Tablettentestgerät nach Anspruch 7, **dadurch gekennzeichnet, dass** der Vorsprung der Transporteinrichtung in Wirkrichtung des Bruchbackens (38) eine Breitenabmessung größer als die Hälfte der Länge oder der Breite der Tablette hat.

9. Tablettentestgerät nach Anspruch 1, **dadurch gekennzeichnet, dass** das Positionierungswerkzeug (58) an seiner dem Anschlag zugewandten Seite eine Nase (60) aufweist, deren untere Begrenzungsfläche (62) bei einer Stelle an einer Stirnfläche (64) des Positionierungswerkzeugs (58) liegt, deren Höhe über der Auflagefläche (6) durch die Dickenabmessung der Tablette bestimmt ist.

## Claims

1. Tablet testing device for testing oblong tablets, coated tablets or similarly-shaped tablets, more particularly for measuring and break-testing such tablets, in corresponding test stations (18, 20), a transport device (2), which comprises a base (4) with a surface (6) onto which the tablets to be tested are placed, as well as a transport means, for transporting the tablets placed on the surface through the test stations (18, 20), whereby the breaking strength measuring device for testing the breaking strength of the tablets comprises a moveable breaking jaw (38) and a counter jaw (24) between which the tablet placed on the surface (6) is broken, a drive mechanism (32) for the breaking jaw (38) and a measuring device (34) for measuring the force required for breaking the tablet, **characterised in that**
in the transport device (2) a moveable stop (26) for arresting the tablet in the breaking strength measuring device (30) is arranged behind the breaking strength measuring device (30), and **in that**
a positioning tool (28; 48; 58) is arranged on the transport means (8) that can be moved towards the stop (26) up to a distance that is determined by the width of the tablet.

2. Tablet testing device in accordance with claim 1, **characterised in that** the transport means (8) is a transporting rake.

3. Tablet testing device in accordance with claim 1 or 2, **characterised in that** the moveable stop has a pivotally arranged flap (26) under the surface (6) which is connected to a drive mechanism that is able to move the flap (26) back and forth between a rest position, in which the flap (26) lies under the surface (6), and a contact position in which it forms the stop **in that** it projects above the surface.

4. Tablet testing device in accordance with claim 1 **characterised in that** the positioning tool (28; 48; 58) is fastened to the transport means (8) in a detachable manner.

5. Tablet testing device in accordance with claim 1, **characterised in that** the positioning tool (48) is arranged to move on the transport means (8) in the direction of action of the breaking jaw (38).

6. Tablet testing device in accordance with claim 1, **characterised in that** the positioning tool (48) is pre-tensioned by spring means against the direction of action of the breaking jaw (38) and can be moved in the direction of action of the breaking jaw (38) by a plunger (52) connected to the moving mechanism of the breaking jaw (38).

7. Tablet testing device in accordance with claim 1, **characterised in that** the positioning tool (28; 48; 58) has a projection on the transport means (8) which extends from the surface (6) up to a height above surface (6) which is higher than the height dimension of the tablet (10, 12, 14; 10', 12', 14').

8. Tablet testing device in accordance with claim 7, **characterised in that** the projection of the transporting device has a width dimension in the direction of action of the breaking jaw (38) that is greater than half the length or width of the tablet.

9. Tablet testing device in accordance with claim 1, **characterised in that** on its side facing the stop the positioning tool (58) has a lug (60) the lower limiting surface (62) of which lies at a point on the end face (64) of the positioning tool (58), the height above the surface (6) of which is determined by the thickness measurement of the tablet.

## Revendications

1. Appareil pour l'essai de comprimés oblongs, de dragées ou de comprimés de conformation analogue, notamment pour la mesure et pour les essais à la rupture de comprimés de ce type dans des postes - d'essai correspondants (18, 20) d'un dispositif de transport (2), comprenant une base (4) avec une surface de dépose (6) sur laquelle les comprimés à tester doivent être déposés, ainsi qu'un moyen de transport, pour le transport à travers les postes d'essai (18, 20) des comprimés à soumettre à l'essai, le dispositif de mesure de la résistance à la rupture des comprimés comprenant pour l'essai de la résistance à la rupture une mâchoire de rupture mobile (38) et une contre-mâchoire (24), pour amener à la rupture mutuelle les comprimés déposés sur la surface de dépose (6), un dispositif d'entraînement (32) pour la mâchoire de rupture (38) et un dispositif de mesure (34) pour mesurer la force nécessaire pour la rupture des comprimés, **caractérisé en ce que**
dans la direction de transport (2) il est disposé derrière le dispositif de mesure de résistance à la rupture (30) une butée mobile (26) pour maintenir les comprimés dans le le dispositif de mesure de résistance à la rupture (30) et **en ce que**
sur le moyen de transport (8) est disposé un outil de positionnement (28 ; 48 ; 58), qui est mobile vers la butée (26) jusqu'à une distance qui est définie par une dimension en largeur du comprimé.

2. Appareil pour l'essai de comprimés 1, **caractérisé en ce que** le moyen de transport (8) est un râtelier de transport.

3. Appareil pour l'essai de comprimés selon la revendication 1 ou 2, **caractérisé en ce que** la butée mobile comporte un clapet (26) logé de façon pivotante sous la surface de dépose (6) qui est relié à un dispositif d'entraînement qui est apte à déplacer de part et d'autre le clapet (26) entre une position de repos dans laquelle le clapet (26) se situe sous la surface de dépose (6) et une position de butée dans laquelle il forme la butée en faisant saillie par dessus la surface de dépose.

4. Appareil pour l'essai de comprimés selon la revendication 1, **caractérisé en ce que** l'outil de positionnement (28 ; 48 ; 58) est fixé de façon amovible sur le moyen de transport (8).

5. Appareil pour l'essai de comprimés selon la revendication 1, **caractérisé en ce que** l'outil de positionnement (48) est logé de façon déplaçable en translation sur le moyen de transport (8), en direction de circulation de la mâchoire de rupture (38).

6. Appareil pour l'essai de comprimés selon la revendication 1, **caractérisé en ce que** l'outil de positionnement (48) est précontraint de façon élastique à l'encontre de la direction de circulation de la mâchoire de rupture (38) et déplaçable en translation dans la direction de circulation de la mâchoire de rupture par un coulisseau (52) accouplé à la direction de déplacement de la mâchoire de rupture.

7. Appareil pour l'essai de comprimés selon la revendication 1, **caractérisé en ce que** l'outil de positionnement (28 ; 48 ; 58) comporte une saillie sur le moyen de transport (8) allant de la surface de dépose (6) jusqu'à une hauteur au-dessus de la surface de dépose (6) qui est plus élevée qu'une dimension en hauteur du comprimé (10, 12, 14 ; 10', 12', 14').

8. Appareil pour l'essai de comprimés selon la revendication 7, **caractérisé en ce qu'**en direction de circulation de la mâchoire de rupture (38), la saillie du dispositif de transport a une dimension en largeur supérieure à la moitié de la longueur ou de la largeur du comprimé.

9. Appareil pour l'essai de comprimés selon la revendication 1, **caractérisé en ce que** l'outil de positionnement (58) comporte sur son côté faisant face à la butée un taquet (60) dont la surface de délimitation inférieure (62) se situe en un point sur une surface frontale (64) de l'outil de positionnement (58) dont la hauteur au-dessus de la surface de butée (6) est déterminée par la dimension d'épaisseur du comprimé.
